# EUROPEAN PATENT APPLICATION

(11) **EP 1 956 508 A2**
(43) Date of publication of application: **13.08.2008**
(21) Application number: 07255040.3
(22) Date of filing: 24.12.2007
(51) Int. Cl.: G06F 19/00

(54) **System and method for implimentation of glycemic control protocols**

(30) Priority: 26.12.2006 US 871856 P; 12.12.2007 US 954887
(71) Applicant: Lifescan, Inc., Milpitas, CA 95035 (US)
(72) Inventor: Harmon, Kirk C., San Ramon California 94582 (US); Furnary, Anthony P. Dr., Portland Oregon 97225 (US); Yu, William, Mountain View California 94040 (US); Telson, Stanley, Lancaster Pennsylvania 17601 (US); Hausman, Paul, San Ramon California 94582 (US)
(74) Representative: Tunstall, Christopher Stephen

(57) **Abstract**

A system for implementing a wide-range of glycemic control protocols (e.g., Tight Glycemic Control protocols) in an automated, semi-closed or closed loop manner thereby eliminating common sources of system error is provided herein. More specifically, the system can include a blood glucose measuring device in communication with a wide-range of databases and/or interfaces thereby allowing for typically independent steps such as patient testing, result determination, protocol adjustment (suggestions as well as actual calculations of new dosages), meal information, scheduling of future testing in light of test results, and administration of medicine to be performed automatically in a semi-closed or closed loop manner. Furthermore, the system provides for ultimate flexibility by allowing a protocol administrator to continuously add, subtract, and/or modify various steps of the protocol while maintaining the integrity and safety of the system. Additionally, methods of implementing such glucose control protocols are also provided herein.

## Description

### RELATED APPLICATION(S)

This application claims priority under 35 U.S.C. §119(e) to U.S. provisional patent application serial no. 60/871,856, filed December 26, 2006, the entirety of which is hereby incorporated herein by reference.

### FIELD OF USE

The present disclosure relates to an integrated system and method for implementing glycemic control protocols (e.g., continuous and/or episodic infusion treatment, oral hypoglycemic therapy, intensive insulin therapy, or any such glycemic control therapy); more specifically, the present disclosure may further relate to semi-closed or closed loop systems and methods for the automated implementation of such protocols.

### BACKGROUND

Hyperglycemia brought on by stress of illness, insulin resistance, and/or insulin deficiency during critical illness may directly or indirectly confer a predisposition to complications such as severe infection, polyneuropathy, multiple organ failure, and even death. Normalization of blood glucose levels with intensive therapy (e.g., administration of insulin or other agent) reduces mortality and morbidity among critically ill patients. For example, in diabetic patients with acute myocardial infarction, therapy to maintain blood glucose at a level below about 215 mg per deciliter (about 11.9 mmol per liter) improves the patient's long-term outcome. In nondiabetic patients with protracted critical illnesses, high serum levels of insulin-like growth factor-binding protein 1, which reflects an impaired response of hepatocytes to insulin, can increase the risk of death. (See Van den Berghe et al, Intensive Insulin Therapy in Critically III Patients, N Engl J. Med 2001; 345:1359-67). In cardiac surgery, intensive insulin therapy can reduce in-hospital mortality, infectious complications, and/or the length of the hospital stay. (See Furnary, A.P., Wu, Y.X.: Clinical Effects of Hyperglycemia in the Cardiac Surgery Population: The Portland Diabetic Project, Endo Pract. Jul-Aug 2006; 12 Suppl 3:22-26).

Present day methodologies for glycemic control most commonly rely on manually transferring blood glucose data obtained from a Food and Drug Administration ("FDA")-approved glucose measuring device to a paper or electronic medical form. The progression and dynamic response of that particular patient's glucose level is assessed by the medical clinician (e.g., a nurse or a physician) caring for the patient, who then integrates that information with a paper-based algorithm, or "protocol" to determine the next insulin-based, or other glucose manipulating intervention to be applied to that particular patient. In the most common scenario, the interaction between the measuring device, the clinician, the protocol, and the insulin action is subject to the interpretive capabilities of the involved clinician.

Less commonly, data from the glucometer is manually entered into computerized algorithm-based devices that are separate systems capable of interfacing with patient information. Such manual transfer of data may lead to significant user error and work-flow inefficiency. In addition, such systems are unable to efficiently account for the large number of variables that must be considered in properly implementing a glycemic control protocol (e.g., time since last glucose measurement, meal information, diabetic or nondiabetic status, cardiac issues, type/amount of medication already being administered, prior test results, current test results, operator/nurse competencies, etc.). Furthermore, the present day methodologies are not able to efficiently account for patient logistics (e.g., patients being admitted to the hospital, patients being transferred from one hospital location to another, and/or patients being discharged and/or re-admitted) when properly implementing a glycemic control protocol.

Thus, there is a need for an integrated system and method capable of implementing any type of glycemic control protocol.

### SUMMARY

Various embodiments of a system and a method of implementing a glycemic control protocol are provided herein. As described below, the presently disclosed embodiments enable a medical professional to determine and store a patient's current blood glucose level at a particular time point. In view of this test result, the system can provide one or any number of treatment steps. For example, the treatment steps can include adjustments to the patient's treatment (e.g., a change in medication, diet, etc.) or the treatment steps can include any of a number of actions (e.g., scheduling a future test, providing various qualitative and/or quantitative patient status indicators, etc.). Further, the system can compare a patient's current test result at a current time point with at least one prior test result taken at a prior time point in order to provide a current treatment step. Additionally, the system can also consider any or all prior treatment steps in determining the current treatment step. In short, the presently disclosed embodiments provide a robust and versatile system and method configured to utilize and manage a wide range of information in order to enhance patient care and treatment outcome.

Various aspects of a system for implementing a glycemic control protocol are provided herein. In one such aspect, the system includes a glucose measuring device (e.g., a hand-help device) configured to determine a patient's current blood glucose level at a current time point. Additionally, the system includes a storage means (e.g., a database, etc.) associated with the glucose measuring device wherein the storage means can be configured to store the patient's current blood glucose level and the current time point. The system can also include processor associated with the glucose measuring device wherein the processor can be configured to implement at least one glycemic control protocol which is configured to provide a treatment step which is at least partially dependent upon the current blood glucose level and the current time point.

As will be described below, the treatment step can be virtually any step suggested or implemented by the system. Also, the system can provide a single treatment step or the system can provide a plurality of such treatment steps. For example, the treatment step can be an adjustment or the treatment step can be an action. Examples of an adjustment include a change medication or a meal adjustment. Regarding the medication adjustment, the adjustment can include a change in a type of medication being administered to the patient or the medication change is an increase or a decrease in an amount or a dosage of the medication being administered to the patient. As will be appreciated by those skilled in the art, the medication can be any medication capable of manipulating the patient's blood glucose level. Also, the adjustment can be suggestion. Examples of an action can include scheduling a future time for a test and/or providing various qualitative and/or quantitative patent status identifiers (which can be provided on a user interface associated with the system).

As indicated, the system can be configured to provide a plurality of treatment steps wherein the treatment steps are at least partially dependent upon the current blood glucose level and the current time point. For example, the plurality of treatment steps can include a first treatment step and a second treatment step. In such an embodiment, the first treatment step can being an adjustment and the second treatment step being an action wherein the action can be at least partially based upon the adjustment. Additionally, the action can be performed automatically in a semi-closed or closed loop manner.

In providing a treatment step, the system can consider factors in addition to the current test result and a corresponding time point. For example, in one embodiment, the storage means can further stores at least one previous patient blood glucose level and a previous time point associated with the at least one previous patient blood glucose level. Additionally, the storage means can be further configured to store a previous treatment step associated with the previous patient blood glucose level and the previous time point associated with the previous blood glucose level. In this embodiment, the system can include a comparison means (e.g., any commonly known comparison means) associated with the glucose measuring device. In use, the comparison means can be configured to compare the patient's current blood glucose level and current time point with the at least one previous patient blood glucose level and the previous time point to provide a glucose rate of change (e.g., an absolute difference, a rate, or a percent) wherein the treatment step is at least partially dependent upon the glucose rate of change.

Adding to the versatility of the system, the glucose measuring device can be configured to distinguish between a plurality of patients such that each patient can be correlated with a corresponding glycemic control protocol. For example, in one embodiment, the glucose measuring device can be configured to communicate with a patient-specific database located on a patient-specific tag (e.g., an RFID tag). Adding to the versatility of the system, the storage system (e.g., database) and/or processor can be located within the glucose measuring device.

In another aspect, a system for implementing a user-definable glycemic control protocol is provided which includes a glucose measuring device configured to determine a patient's blood glucose level at a current time point. Additionally, the system includes a memory system having a storage means wherein the memory system can be configured to communicate with the glucose measuring device and the storage means can be configured to store the patient's current blood glucose level and the current time point. Additionally, the system can include a processor configured to communicate with the glucose measuring device. The processor can be further configured to implement a user-definable protocol wherein the use-definable protocol can be configured to determine a treatment step based at least partially on the patient's current blood glucose level and the current time point. Similar to above, the memory system and/or the processor can be located within the glucose measuring device.

As indicated, the system can allow an authorized operator (e.g., a protocol administrator) to modify the protocol as desired. For example, in one embodiment, the system can include an editor in communication with the processor wherein the editor can be configured to modify the glycemic control protocol in response to instructions from a protocol administrator.

In some embodiments, the system can be configured to ensure that authorized operators are utilizing the system (e.g., performing tests and/or modifying the protocol.) For example, the glucose measuring device can be configured to receive an operator identifier (e.g., an identification number) prior to use wherein the storage means can be configured to associate the operator identifier with the current blood glucose level and the current time point. Also, the system can further require a second operator identifier (e.g., the second operator identifier being distinct from the first operator identifier) associated with a second operator prior to use. In some embodiment, the system can require the second operator identifier prior to the incorporation of any adjustments and/or actions into the glycemic control protocol.

Various aspects of a method of implementing a glycemic control protocol are also provided herein. In one such aspect, the method includes loading a glycemic control protocol onto a glucose measuring device wherein (similar to above) the glycemic control protocol can be configured to provide at least one treatment step (e.g., an adjustment and/or an action) based at least partially on a patient's blood glucose level measured at a specific time point. Next, the method can include measuring a patient's current blood glucose level with the glucose measuring device, and associating the patient's current blood glucose level with a current time point. The method also can include providing an treatment step which is at least partially determined by the glycemic control protocol. Additionally, the method can further include storing the patient's current blood glucose level, the associated time point, and the treatment step in a database in communication with the glucose measuring device.

In some embodiments, the method can also include comparing the patient's current blood glucose level and current time point with at least one prior blood glucose level determined at a prior time point so as to provide a glucose rate of change, and providing a treatment step which is based at least partially dependent upon the glucose rate of change. In such an embodiment, the treatment step can determined by the glycemic control protocol's interpretation of the rate of change and a prior action. Optionally, the method can also include a step for determining if the patient's current blood glucose level is a valid test result.

In yet another aspect, a method for implementing a glycemic control protocol is provided which includes providing a glucose measuring device and also providing a memory system having a storage means containing a plurality of glycemic control protocols. Additionally, the method can include providing at least one patient, and associating the at least one patient with a patient-specific glycemic control protocol wherein the patient-specific glycemic control protocol can be selected from the plurality of glycemic control protocols contained within the storage means. The method can further include measuring the patient's current blood glucose level, associating the patient's current blood glucose level with a current time point, and providing a treatment step which is determined by the patient-specific glycemic control protocol. Optionally, the method can also include loading the patient-specific glycemic control protocol onto the glucose measuring device.

The method can also include providing a plurality of patients, and further associating each patient of the plurality of patients with a patient-identification tag wherein the patient identification tag includes a storage means configured to store a plurality of patient-specific information. In such an embodiment, the glucose measuring device can be configured to communicate with the patient's patient-identification tag. Also, the method can further include (e.g., following the associating step) modifying the patient-specific protocol thereby providing a user-definable protocol.

### BRIEF DESCRIPTION OF THE DRAWINGS

The present disclosure will be more fully understood from the following detailed description taken in conjunction with the accompanying drawings, in which:
FIG. 1A is an illustrative representation of possible components of an embodiment of the presently disclosed system;
FIG. 1B is another illustrative representation of the components of the embodiment of FIG. 1A;
FIG. 2A is an illustrative representation of various embodiments of the presently disclosed system being incorporated into a hospital's network;
FIG. 2B is a view of an embodiment of a glucose measuring device docking station capable of coupling the glucose measuring device to other components of the system;
FIG. 3 is a flow chart of an overview of the basic steps of an embodiment of the presently disclosed system;
FIG. 4 is a flow chart showing three general stages of an embodiment of the presently disclosed system;
FIG. 5 is a flow chart of an exemplary embodiment of a Pre-Testing Stage of the presently disclosed system;
FIG. 6 is a flow chart of an embodiment of a quality control step performed upon activation of the glucose measuring device;
FIG. 7 is a flow chart of an exemplary embodiment of a Patient Testing Stage of the presently disclosed system;

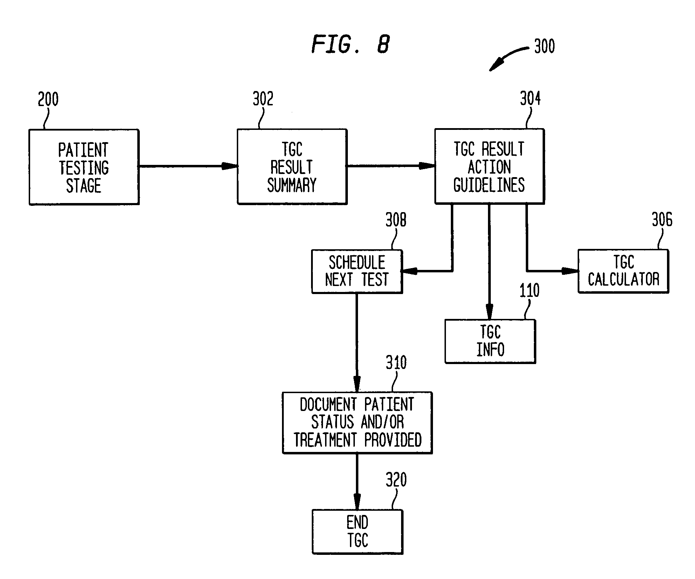

FIG. 8 is a flow chart of an exemplary embodiment of a Post-Testing Stage of the presently disclosed system;
FIG. 9 is a flow chart of an alternative embodiment of a Operator-Confirmation stage;
FIG. 10 is a flow chart of an exemplary embodiment of the presently disclosed system wherein a desired glycemic control protocol has been loaded on a OneTouch® Flexx TGC Meter^{™};
FIGS. 11A-11U are various examples of user interface screens which can appear on the glucose measuring device while utilizing the system of FIG. 10; and
FIGS. 12A-12R are further examples of user interface screens which can appear on the glucose measuring device while utilizing the system of FIG. 10.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. Those skilled in the art will understand that the devices, systems, and methods specifically described herein and illustrated in the accompanying drawings are non-limiting exemplary embodiments and that the scope of the present disclosure is defined solely by the claims. The features illustrated or described in connection with one exemplary embodiment may be combined with the features of other embodiments. Such modifications and variations are intended to be included within the scope of the present disclosure.

Various embodiments of a system for implementing a wide-range of glycemic control protocols in an automated, semi-closed or closed loop manner thereby eliminating common sources of system and/or interpretive error are provided herein. More specifically, the presently disclosed embodiments provide a blood glucose measuring device in communication with a wide-range of databases and/or interfaces thereby allowing for typically independent steps such as patient testing, result determination, protocol adjustment (suggestions as well as actual calculations of new dosages), meal information, scheduling of future testing in light of test results, and administration of medicine to be performed manually or automatically in a semi-closed or closed loop manner. Furthermore, the system provides for ultimate flexibility by allowing a protocol administrator to continuously add, subtract, and/or modify various steps of protocol while maintaining the integrity and safety of the system. Thus, the system can implement a user-definable protocol as opposed to being limited to a single (or a pre-determined) protocol. In short, the presently disclosed system and method can be utilized to provide hospitals, various medical facilities, and/or patients themselves with a powerful technology capable of best optimizing resources (i.e., time and money) as well as providing improved patient care by real-time interpretation of glucose testing, real-time comparison and/or interpretation of such results in relation to previous result and/or previous courses of action, and by the elimination of common errors (e.g., incorrect data input and/or interpretation).

As will be appreciated by those skilled in the art, any glycemic control protocol can be implemented with the presently disclosed system. As will be described, the system can also implement any of a plurality of protocols as opposed to being limited to a single pre-determined protocol. Additionally, the system can allow for a protocol administrator to modify the protocol thereby allowing for a user-definable protocol. In an exemplary embodiment, the glycemic control protocol is a Tight Glycemic Control ("TGC") protocol. While various examples below describe TGC protocols, those skilled in the art will appreciate that any other such glycemic control protocol is within the spirit and scope of the present disclosure. Similarly, in some embodiments, the system can control the amount and type of medication being delivered (e.g., intraveneously, orally, trans-dermally, trans-bronchially, inhalation, absorbed trans-mucosal, transnasally, subcutaneously, etc.) to a patient in order to move (or maintain) a patient's blood glucose level to within a target range. Those skilled in the art will also appreciate that any medication capable of manipulating blood glucose levels can be utilized by the presently disclosed system. In an exemplary embodiment, the medication is insulin. In other embodiments, the medication can include glucose, an insulin analogue, glucagon, any insulin sensitizing agent, a sulfonourea, thiazolidinedione, an amylin or amylin mimetic agent, a glucagons suppressor, an insulin secretagogue, an incretin or incretin mimetic, a biguanide, a GLP-1 analogue, a DPP inhibitor, a DPP inhibitor, or any combination thereof Thus, the presently disclosed system can implement any glucose control protocol capable of utilizing any of a wide variety of medication capable of manipulating a patient's blood glucose levels thereby maintain the level within a target range.

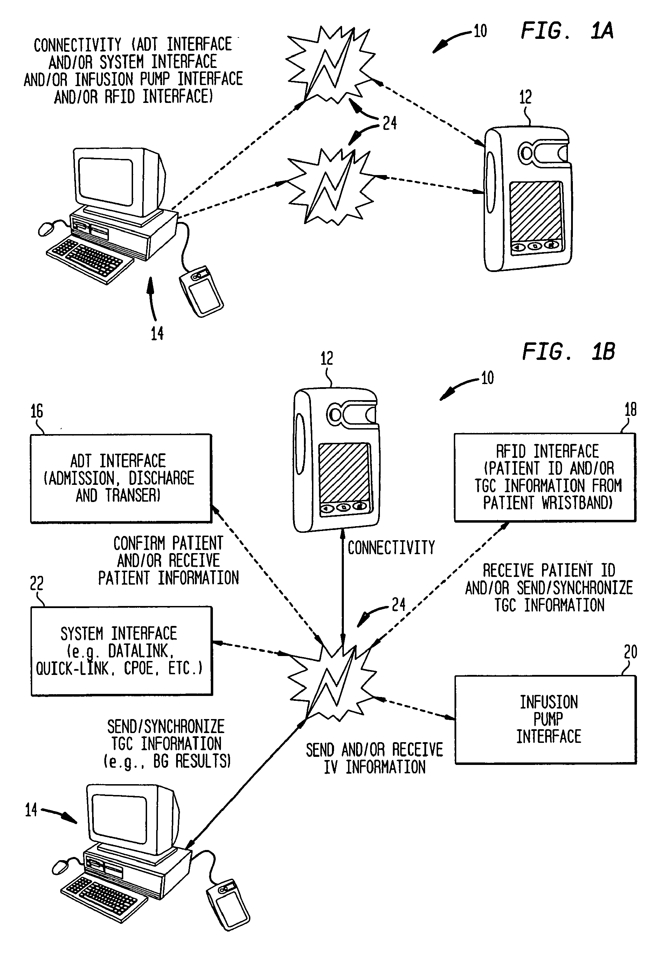

Referring now to FIG. 1A, the system 10 can include a glucose measuring device 12 and a computer workstation 14 (e.g., a desktop computer, a personal data assistant, etc.) in communication 24 with any number of interfaces in order to provide an integrated network. FIG. 1B represents the connectivity of these various interfaces. As shown, the interfaces can include any type of information as desired by the protocol administrator. In an exemplary embodiment, the system 10 includes an ADT interface 16, a radiofrequency identification ("RFID") interface 18, an Infusion Pump Interface 20, and a System Interface 22. The ADT Interface 16 can include any type of information related to patient admission-discharge-transfer information. Next, the RFID Interface 18 can include any type of information related to patient identification (e.g., an identification number) and/or TGC information (as related to the specific patient or merely general information). Further, the Infusion Pump Interface 20 can be configured so that the system 10 is capable of delivering medicine to the patient in response to a certain patient test (e.g., episodic and/or continuous BG testing). Once again, such an interface further eliminates any potential user error. Finally, the System Interface 22 may utilize any type of data exchange protocol interface in order to exchange data. For example, such a data exchange protocol interface can include any of DataLink, Quick-Link, CPOE, LIS, etc.

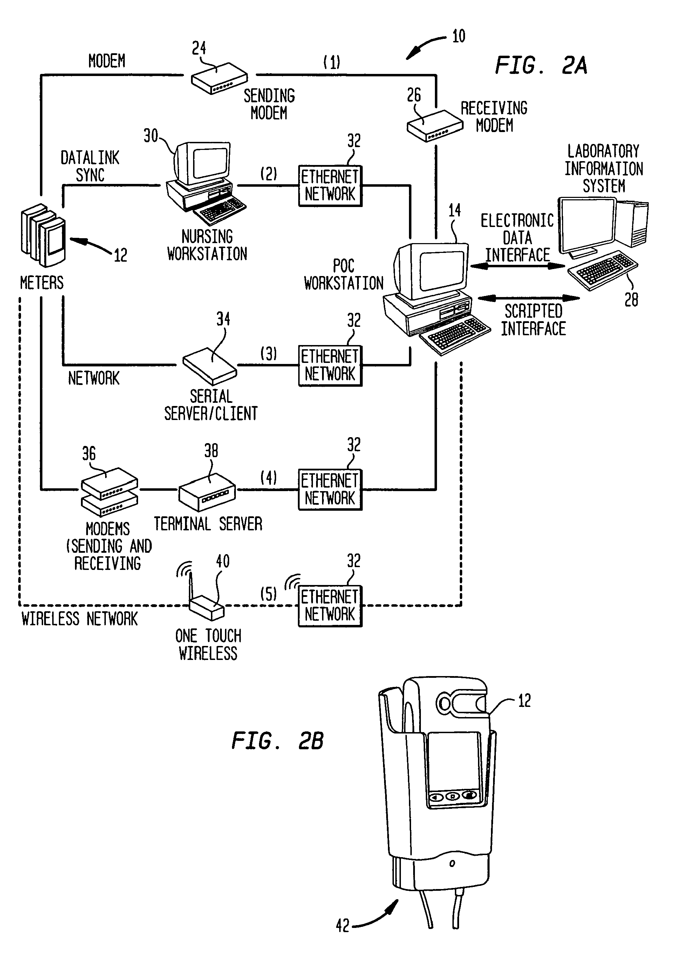

As shown in FIG. 2A, various pathways can allow for the glucose measuring device 12 to be brought into communication with a point-of-care ("POC") workstation 14 and/or Laboratory Information Management System 28, as well as all other elements of the system. The following are merely provided as examples and are in no way meant to limit the scope of the present disclosure. In the first pathway, "the Modem pathway", the glucose measuring device 12 can be coupled to a sending modem 24 which in turn can be coupled to a receiving modem 26. Further, the receiving modem 26 can be in communication with a POC Workstation 14. Next, the Workstation 14 can be coupled to a Laboratory Information System 28 via an Electronic Data Interface as well as a Scripted Interface. In the second pathway, "the DataLink Sync Pathway", the glucose measuring devices 12 can be coupled to a nursing workstation 30 which is linked to the POC Workstation 14 via an Ethernet Network 32. The remaining three examples provide other various means of connecting the glucose measuring devices 12 to the Ethernet 32 such as via a Serial Server/Client 34 (pathway 3; "the network pathway"); via Sending/Receiving Modems 36 and a Terminal Server 38 (pathway 4; "the Multiple-Receiving Modem"); and via a One Touch Wireless 40 unit (pathway 5; "the Wireless Network"). FIG. 2B is an example of a docking port 42 for the glucose measuring device 12. In this example, when docked, the glucose measuring device 12 can be configured to connect and/or synchronize with the network. In these various embodiments, the system 10 can store various types of information via any commonly known storage means. For example, the storage means can include flash memory, databases, battery back-up RAM, etc. wherein the information may be stored locally or remotely (i.e., wirelessly).

Looking at the glucose measuring device 12, the device 12 can be any device capable of storing and/or implementing a glycemic control protocol in order to accurately determine a patient's current blood glucose ("BG") level (an accurate test being within about +/- 15% or better relative to a reference glucose measuring system). Furthermore, the glucose measuring device 12 can be configured so as to store various types of patient and/or protocol information (e.g., previous test data, meal information, etc.). Furthermore, the glucose measuring device 12 can be configured to perform various comparisons and establish various trends between current and prior test data, suggest changes to current protocols (e.g., changes is medication and/or diet) based on test data and or previous treatments, calculate changes to protocol based on suggested changes, and/or schedule future testing in light of the above information. In an exemplary embodiment, the glucose measuring device 12 is a OneTouch® Flexx^{™} with TGC meter available from LifeScan, a Johnson & Johnson company. The various functions of the glucose measuring device 12 and system 10 will be described in detail below.

The glucose measuring device 12 can include a user interface capable of displaying certain data and/or information to the user (e.g., healthcare professional, patient, etc.). In addition, the glucose measuring device 12 can include various buttons and/or links allowing the user to input information as well as navigate from one screen to the next in order to view different types of information. Such devices are commonly used and therefore various embodiments of such devices would be readily apparent to those skilled in the art.

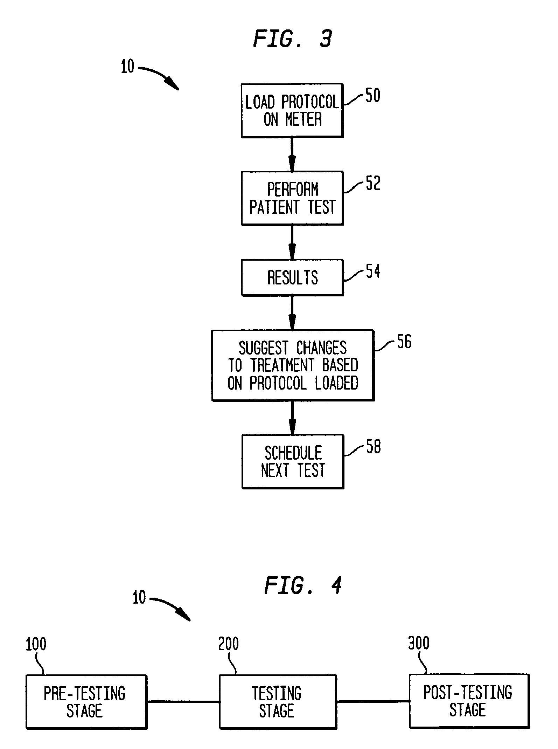

As will be apparent from the discussion below, the implemented glycemic control protocols can be as simple or as complex as desired by a protocol administrator. While the system can utilize virtually any glycemic control protocol, FIG. 3 provides an example of steps typically performed in a Tight Glycemic Control ("TGC") protocol. For example, in a preliminary step, a protocol administrator can load a desired protocol on the glucose measuring device 12 (Step 50). As stressed below, the present system 10 is sufficiently robust and flexible so as to allow the administrator to implement any protocol as deemed necessary and/or desirable or the administrator can implement different protocols for different glucose measuring devices (e.g., different protocols for glucose measuring devices located in different areas of the hospital). Additionally, the protocol administrator can modify the protocol thereby allowing for a user-definable protocol. Typically, a glycemic control protocol will initially establish a target range for a patient's BG level such that all future testing may be compared to this target in order to determine a patient's status. The desired target BG range may be any range (and be defined by any unit of measure) as desired by the protocol administer. The ranges may depend of various factors such as the patient's condition, the type of procedure the patient may be undergoing, etc. As discussed above, these ranges may also dependent on the standard of care and/or user experience with the protocol. In an exemplary embodiment, the target range is from about 70 mg/dL to about 150 mg/dL, from about 80 mg/dL to about 120 mg/dL, more preferably completely in the internationally defined euglycemic range of from about 70 mg/dL to about 110 mg/dL. Those skilled in the art will appreciate that any such range is within the spirit and scope of the present disclosure.

Once the protocol is loaded, the system can proceed to a patient test (Step 52). The test can be any test capable of accurately determining a patient's current BG level. Typically, the test includes obtaining a patient sample (e.g., a blood sample) and delivering the sample to a glucose measuring device 12 thereby determining the current BG level at a current time point. As will be shown in detail below, the glucose measuring device 12 can be configured so as to allow for the current test result and current time point to be directly utilized by the system 10 thereby eliminating any need for the information to be manually transferred by a user to a distinct, second system as this manual transfer step can be a significant source of error.

Next, the system 10 can allow for a series of results to be produced from the patient test (Step 54). The results can include a determination of the current BG level, a comparison of the current BG level to the desired target range, and/or a comparison of a previous patient result at a previous time point compared to the current BG level at the current time point. Additionally, the system 10 can determine how dramatically the BG level is increasing/decreasing (i.e., rate of change). In addition, the results may include a qualitative status identifiers such as "High" (current BG level above target range), "Low" (current BG level below target range), "Rising" (current BG level higher than previous BG level), etc. As will be apparent to those skilled in the art, the system 10 can be configured to provide various types of quantitative and/or qualitative results as desired by the protocol administrator.

Next, the presently disclosed system can suggest changes or modifications to the current treatment in order to allow a patient's BG level to return and/or remain within the desired BG range (Step 56). For example, in light of the testing results mentioned above, the system 10 can suggest a change in medication (e.g., from insulin to glucose or vice versa), the additional administration of a medicine, or a change in dosage of medication (e.g., increase administration of insulin by X amount/percent for Y hours, or addition of an alternative glucose controlling medication other than insulin). Alternatively, the system 10 can suggest no change in therapy or the system 10 may stop the administration of medication until a patient's BG level has increased by a certain amount (as in the case of a hypoglycemic patient). In these cases, the system can automatically implement a "Restart State" which is capable of being configured to a patient's being mildly or, alternatively, severely hypoglycemic. Once the appropriate changes are determined, the system 10 can perform the necessary calculation to modify the current treatment to the new treatment. For example, if the protocol programmed into the system suggests a 20% increase in medication (e.g., insulin), the system can calculate and inform the user of the new amount of such medication to be administered. Once again, the semi-closed or closed loop re-calculation step substantially eliminates user error from this step (e.g., an incorrect user calculation or an error resulting from configuration of IV Pump for insulin infusion).

Finally, the system 10 can provide an action such as suggesting a date and time for the next patient test (Step 58). The date and time can be determined in light of the currents results, in light of any suggested adjustments to protocol discussed above, in light of a comparison between current test results at current time and previous test results at a previous time, etc. For example, if a patient's BG level is above or below the desired range or if significant adjustments have been implemented in a patient's therapy (e.g., increase/decrease in medication), the next test can be scheduled sooner as opposed to a patient who is stable or where no adjustments in dosage and/or therapy are suggested. Therefore, the system can be configured to efficiently utilize hospital time and technology. As will now be shown, each of the steps discussed above may be broken down into numerous sub-steps in order to provide the protocol administer with a robust system 10 capable of implementing any desired protocol for any type of patient (i.e., diabetic, nondiabetic, cardiac, etc.).

In general, as represented in FIG. 4, the presently disclosed system can be divided into three stages: a Pre-Testing Stage (100), a Testing Stage (200), and a Post-Testing Stage (300). Please note, this break-down is merely presented for ease of discussion. As will be readily apparent to those skilled in the art, the various stages are inter-related and produce an integrated system. For example, many of the steps occurring in the Pre-Testing Stage (100) may be moved or repeated in one of the other stages (200, 300) if so desired by the protocol administrator. As such, these stages (100, 200, 300) are now described to provide an example of the various steps available to the protocol administrator when implementing a protocol with the presently disclosed system 10.

### Stage 1: Pre-Testing Stage

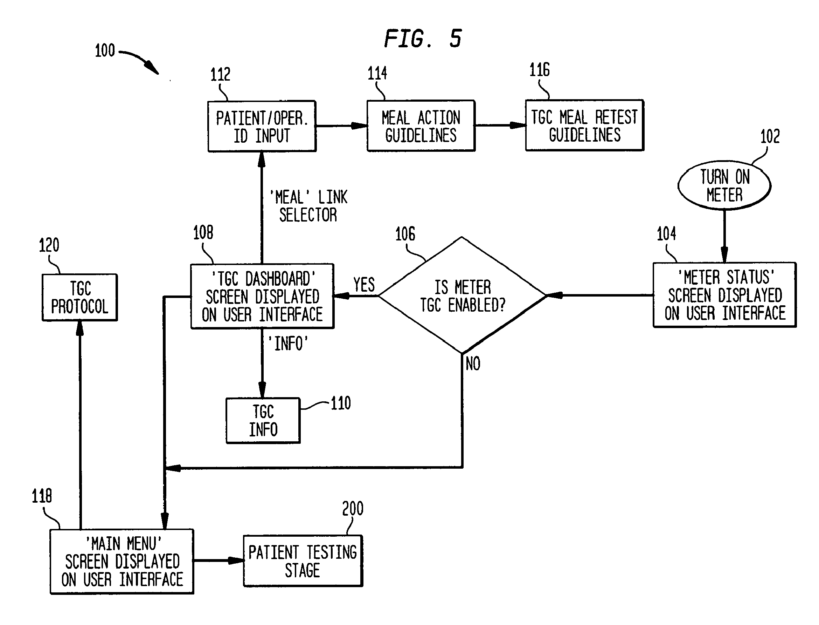

In general, the Pre-Testing Stage (100) can be considered to include all steps from powering on the glucose measuring device (Step 102) up to the beginning of the Patient Testing Stage (200). As shown in the exemplary embodiment of FIG. 5, the system 10 can be initiated by powering on the glucose measuring device 12 (Step 102). Once activated, the glucose measuring device 12 can be configured to display a Meter Status screen (Step 104) capable of displaying various types of basic information to the user. For example, the Meter Status screen (Step 104) can display a glucose measuring device identification number, the current date and time, battery strength, an indication of when glucose measuring device 12 is due for a future QC procedure, a name and/or identification number of protocol(s) loaded on the glucose measuring device 12, an indication of whether or not the glucose measuring device 12 has been enabled for TGC-related testing, etc..

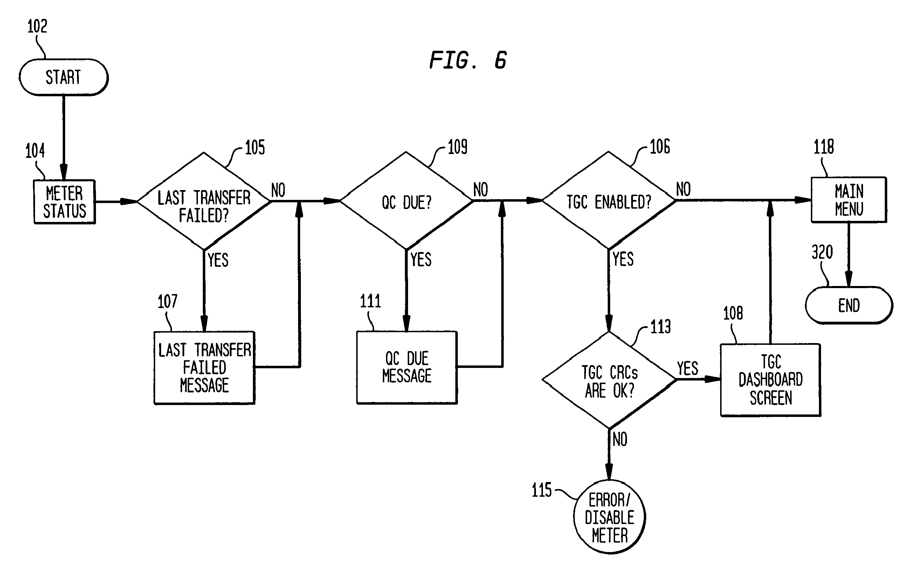

In addition to such basic information, the Meter Status screen (Step 104) can also be configured to inform a user of any corruption associated with the protocol and/or the glucose measuring device 12. For example, prior to performing any additional step(s), a cyclical redundancy check ("CRC") for the implemented TGC protocol and any TGC patient information coupled to the glucose measuring device 12 can be checked for corruption. If any corruption is detected, an appropriate error message (e.g., "Last Data Transfer Failed", "QC Failed", or "TGC Protocol Corrupted", etc.) can be displayed and the glucose measuring device 12 disabled from patient testing. Updating the glycemic control protocol (as will be discussed below) shall then be required using, for example, the TGC Protocol Editor and Transfer tool, to fix the corruption. If any glycemic control patient information record is corrupted, then updating the glycemic control protocol shall also clear the corrupted patient information record. FIG. 6 provides an exemplary embodiment of the above-described corruption check. As shown, the Meter Status screen (Step 104) may be linked to a "Last Transfer Failed?" step (Step 105). If 'yes', the glucose measuring device 12 prompts a failure message (Step 107). If 'no', the glucose measuring device 12 asks is "QC Due?" (Step 109). If 'yes', glucose measuring device 12 can prompt "QC Due" Message (Step 111). If 'no', the system 10 can be configured to ask if the glycemic control protocol (e.g., TGC) is enabled (Step 106). If 'no', the glucose measuring device 12 does not contain the protocol and the user is directed to Main Menu (Step 118) in order to select non-TGC related tests. If 'yes', the glucose measuring device 12 asks if TGC CRCs are OK? (Step 113). If 'no', system 10 can prompt an error message and/or disable the glucose measuring device 12 in order to prevent future use (Step 115). If the answer is 'yes', the user may be directed to a TGC Dashboard (Step 108) and the system 10 can run to completion (Step 320). The Main Menu (Step 118) and TGC Dashboard (Step 108) will be discussed in detail below. As will be appreciated by those skilled in the art, any of a number of alternative calibration steps or procedures are within the spirit and scope of the present disclosure.

Returning to FIG. 5, after reviewing the information provided by the Meter Status screen (Step 104), a user may select to continue to a TGC Dashboard screen (Step 108) which can be configured to present patient information for all those who have recently undergone glycemic control testing. As indicated by the flow chart, prior to delivering the user to the TGC Dashboard (Step 108), the system 10 will determine whether of not the glucose measuring device is "TGC Enabled?" (Step 106). If yes, the user will be directed to the TGC Dashboard (Step 108) and can proceed as will be explained below. If the glucose measuring device 12 is not TGC Enabled, the user can be directed to a Main Menu (Step 118) and proceed with non-TGC testing. As such, the glucose measuring device can be configured to perform both TGC and non-TGC testing protocols.

In general, the TGC Dashboard (Step 108) can include any type of patient information the protocol administrator may feel would be necessary and/or helpful to the user treating the patient. For example, the TGC Dashboard (Step 108) can be configured to identify any patient who has been tested with the glucose measuring device 12 in the last hour, the last 5 hours, the last 12 hours, the last day, the last 2, 3, 4, 5, 10 days, etc. Alternatively, the system 10 may be configured to identify the last 10, 20, 30, 40 , 50, 100, etc. patients who have been tested by the glucose measuring device 12. Further, the TGC Dashboard (Step 108) can be configured to link various types of patient data to each patient identification number. For example, the TGC Dashboard (Step 108) may include a patient identification number, the time and date of the next scheduled test, and the time-date-result of a prior test, etc. Also, the Dashboard (Step 108) may include time and date of patient's last meal as well as any comment(s)/note(s) regarding the patient, the previous test, the treatment protocol, treatment suggested and/or the actual treatment performed, etc.

Additionally, the TGC Dashboard (Step 108) can include a link to "TGC Information" (Step 110) and a link to "Meal Information" (Steps 112, 114, 116). As will be seen below, TGC Information (Step 110) can include any additional information regarding the patient and/or TGC protocol as deemed necessary and/or desirable by the protocol administrator. As such, the feature provides added flexibility to the system 10. Regarding the "Meal Information" link, selecting the "Meal" link can prompt a user to input an operator and patient ID (Step 112). This step will be discussed in detail below as related to similar steps associated with patent testing (See Steps 202 and 204). In short, the identification step (Step 112) allows for the protocol administrator to ensure that authorized and trained personal are operating the glucose measuring devices (and thus performing the testing). In addition, the patient ID link serves to link any patient testing to any stored information relating to the patient but also ensures proper patient identification. Once the identification numbers are provided (Step 112), a user can be directed to a Meal Action Guidelines step (Step 114)). The Meal Action Guidelines step (Step 114) can provide a user with all meal information related to a specific patient. For example, such meal information can include time-date-content (i.e., amount of carbohydrates) of a prior meal, time-date-content of a next meal, etc. Furthermore, a user can select to continue to a TGC Meal Retest step (Step 116) wherein the user can schedule a retest time based on meal consumed and/or record when meals are consumed. Therefore, these steps (Steps 112, 114, 116) allow for a user to monitor and link a patient's dietary habits to any other testing and/or patient data/information.

Following a review of any/all patient information provided by the TGC Dashboard (Step 108), the system 10 can be configured to direct a user to a Main Menu screen (Step 118). In general, the Main Menu screen (Step 118) can be configured by the protocol administrator to include any number and/or type of action items as desired. For example, the action items may include "Perform QC Test", "Review Patient Results", etc. In the exemplary embodiment of FIG. 5, the Main Menu screen (Step 118) allows for: Patient Test and/or Protocol Setup (Step 120). The Patient Test option will be discussed in detail in the next stage. As for Protocol Setup (Step 120), this step allows the administrator to modify the protocol as desired. For example, the administrator may add, remove and/or modify steps as desired by means of an TGC Editor and Transfer tool thereby allowing the system to implement a user-definable protocol.

### Stage 2: Patient Testing Stage

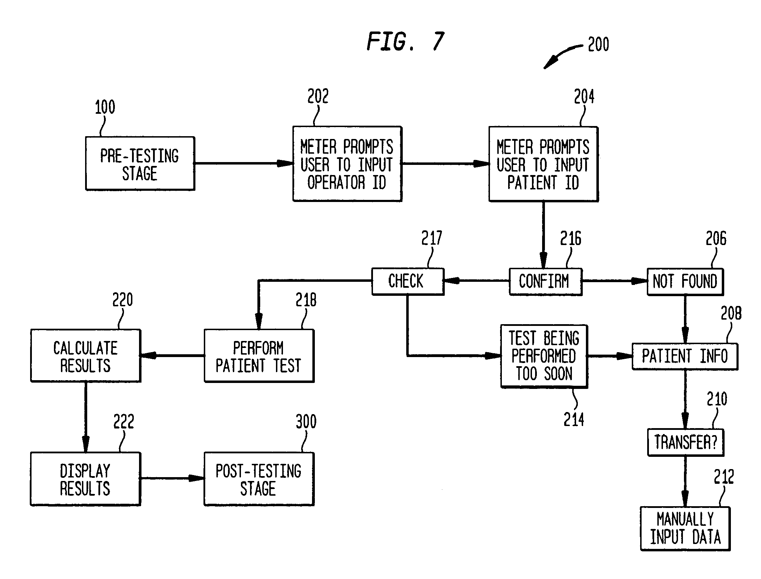

At this stage (200), the system 10 can perform a patient test in order to accurately determine a patient's current blood glucose ("BG") level via episodic and/or continuous blood glucose testing. As stated above, the test can include any glucose measuring device 12 configured to accurately determine the patient's current BG level at a current time point. In an exemplary embodiment, the device 12 is the OneTouch®Flexx™ glucose measuring device. In addition to performing the test (Step 218), the stage (200) can also include any other steps as desired by the protocol administrator. FIG. 7 provides an exemplary embodiment of a Patient Testing Stage (200).

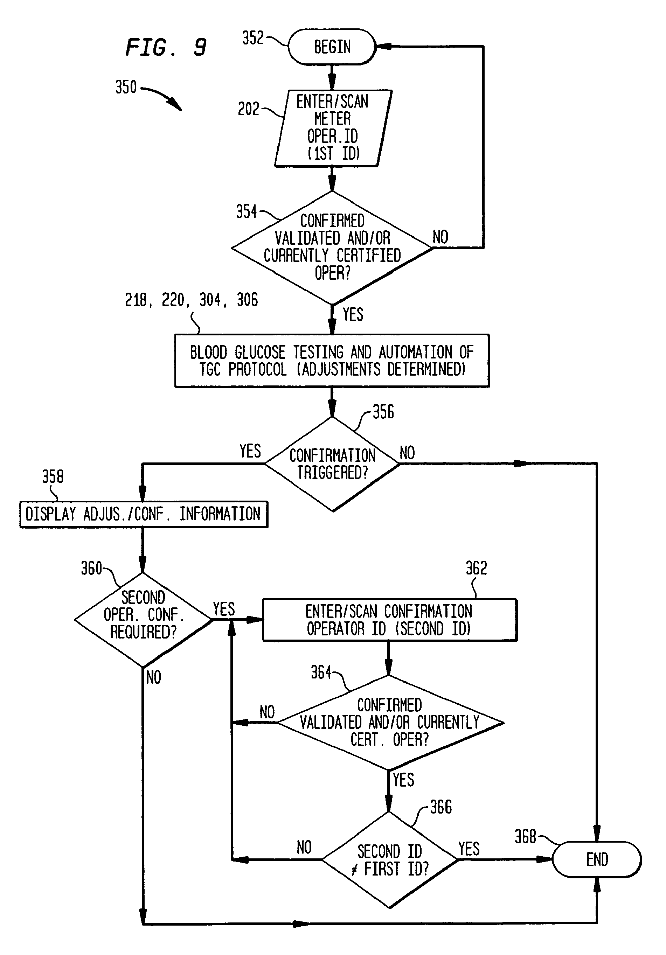

Upon entering the Patient Testing Stage (200), a user can be prompted to input an operator identification number (Step 202). As briefly mentioned above, requiring input of an operator identification number (Step 202) can allow the protocol administrator to ensure the glucose measuring device 12 is being used by an authorized/trained user. In addition, the authorization step (Step 202) can produce a record of which operator is performing which test and treatment to which patient. In one embodiment, the protocol can be configured to require multiple operator identification numbers thereby requiring a confirmation check for the user (as described in detail below in relation to FIG. 9). As for entering the information into the glucose measuring device 12, the identification number can be manually entered or can be scanned into the glucose measuring device 12 from the user's personal identification card (or any other source). Once the operator identification information is successfully entered (Step 202), the system 10 can prompt the user to enter the patient identification number (Step 204). Once again, this information can be manually entered or scanned into the glucose measuring device (e.g, by a radiofrequency identification ("RFID") element on a patient wrist-band or other identification tag). Furthermore, the protocol administrator can require a confirmation of the patient's identification number (Step 216) (manually or automated via an ADT interface). Once successfully entered, the glucose measuring device 12 can link any information resulting from the current test to any patient information stored or in communication with the system 10.

Following input of user/patient identification numbers, the system 10 can include a confirmation step (Step 216) wherein the system can determine if the patient identification number is recognized. If not found, the system 10 can inform the user that the patient's identification number is not recognized (Step 206) by the system 10. This can result for several reasons. First, the patient may have been recently transferred and the information was not successfully transferred to the system 10 and/or glucose measuring device 12. Also, the system 10 may determine that the previous test is too old for use in the current protocol. In these cases, the protocol may direct a user to a Patient Information Menu (Step 208) which allows for the user to input some information regarding the patient (e.g., Insulin Dependent, Non-Insulin Dependent, non-TGC patient, Patient Transfer, etc.) in order to build a preliminary patient record. If Patient Transfer (Step 210) is selected, the user may be allowed to manually enter (Step 212) a prior BG level from the patient's records into the system 10. In cases where data is being manually entered (Step 212), the protocol can require confirmation of the data entry by a second user.

If the patient identification number is recognized, the system 10 can include a check step (Step 217) wherein the system 10 can determine if there is any reason not to continue testing. For example, the system can include a safety precaution wherein the system 10 may prompt the user that a minimum time period has not been achieved since the last test (Step 214) and/or treatment was performed. This feature is meant to prevent cases where a first user performs a test/treatment and a second user, not realizing the test/treatment has already been performed, attempts to perform a second test/treatment. In this case, the protocol administrator can allow for the user to override the error message if desired (which may once again require confirmation from a second user).

Once the operator and patient identification numbers have been successfully entered and confirmed (if necessary), the user may be delivered to the patient testing step (Step 218). As stated above, the system 10 can include any steps capable of accurately measuring the patient's current blood glucose level as well as allowing for a user to verify if last test result was valid (last test result allows for future comparison to current test result). Typically, the protocol can include delivering a patient sample (i.e., blood) to the glucose measuring device 12 via a delivery device (such as a test strip, direct contact with blood from a sterile tubing attached to the patient, an implanted sensor, etc.). After the test, a current BG level for the patient can be determined and automatically utilized by the system 10 to determine various patient results (e.g., actions and/or adjustments) which can be displayed at the Patient Result screen (Step 222).

In determining the patient results (Step 220) and subsequent steps (scheduling a subsequent test, adjusting medication and/or diet, etc.), the system 10 can be configured to perform any type and/or number of comparisons as required by the implemented protocol. Further, the system 10 can be configured to identify any trends in testing (Step 220). For example, the current BG level at the current time point can be compared to the desired range in order to determine if the current level is high, low or within range. Also, the current level may be compared to critical levels (a level above or below a normal range) to determine if the current level is critically high or low and provide user instructions and/or guidelines (providing such instruction may be important is these critical cases). Further, the protocol can be configured so as to allow for the current BG level at the current time to be compared with the patient's prior (one or more) BG levels taken at a prior time so as to determine if the levels are rising, falling or remaining stable. In addition to performing the desired comparisons, the protocol may be configured so as to provide qualitative information (i.e. status identifiers) such as "High", "Low", "Critically High", "Critically Low", "Levels Rising (fast/slow)"; "Levels Falling (fast/slow)", "Stable", "Call Doctor!", etc. or any other non-worded symbols representing these informative status identifiers. Additionally, based on any combination of these variables, the system can provide various steps which can include any number of actions (e.g., scheduling a time point for a subsequent test, calling for an intervention, etc.) and/or the steps can include various suggested adjustments to the treatment protocol (e.g., adjustments in medication, diet, etc.). For example, the results can include instructions to perform a retest (a "Restart Mode") if an error was recognized or if a patient's level is far from the desired level. In one embodiment, the patient may be identified as hypoglycemic and prevented from obtaining any further medication (e.g., insulin) until the test result rises to a specified level. In this case, the results display interface (Step 222) may be configured to specifically address this specific condition (e.g., utilizing a qualitative message such as "Level too low; No insulin!") These identifiers along with the actual test data can be displayed to the user at the Patient Results Display Screen (Step 222). Following a review of the data, the system 10 can be configured to direct the user to the Post-Testing Stage (300).

### Stage 3: Post-Testing Stage

Entering the Post-Testing Stage (300), the system 10 can be configured to deliver a user to a Results Summary interface (Step 302) which allows a user to review various types of patient-related information such as date-time-result of the current test, date-time-result of any prior test, any meal information, and/or any comment(s)/note(s) related to the patient (e.g., status identifiers, previous treatments performed, etc). Additionally, the Result Summary interface (Step 302) can include a link to the TGC Information page (Step 110). As discussed above, the TGC Information page (Step 110) can provide any additional patient and/or protocol information as desired by the protocol administrator. After reviewing the information provided at the Results Summary interface (Step 302), the system 10 can direct the user to the TGC Result Action Guidelines step (Step 304).

The TGC Result Action Guidelines step (Step 304) can be configured to suggest various changes to the treatment ("adjustments") in light of the various test data determined above. Typically, the changes are suggested/required in order to return the patient's BG level to within the desired range and/or to maintain the level within the desired target range. For example, the protocol may suggest changing medication (e.g., from insulin to glucose or vice versa) or adding/subtracting medications to/from a patient's therapy. Furthermore, the system 10 can be configured to suggest increasing/decreasing the medication being administered to the patient (for example, increase insulin administration by X amount/percent for Y hours or decrease insulin by Z units/h). Furthermore, the suggestion may be meal adjustments (i.e., decrease portion size, amount of carbohydrates, etc.). It will be apparent to those skilled in the ant that any of a wide range of other such adjustments are within the spirit and scope of the present disclosure.

If changes in dosage are suggested, the system 10 can be configured to include a TGC Calculator (Step 306) configured to perform the actual calculation required to modify the treatment protocol to the new, suggested/required treatment. In a further embodiment (not shown on flow chart), the system can be configured to trigger the actual administration of the medication after making the suggested/required change (e.g., the system 10 being in communication with an IV pump). These steps can substantially eliminate any calculation errors resulting from a user's attempts to determine the required increase/decrease in dosage.

Following a review of any suggestions provided at the TGC Result Action Guideline (Step 304) and implementing any desired suggestions by means of the TGC Calculator (Step 306), the system 10 can proceed to the step of Scheduling Future Tests (Step 308). At this step (Step 308), the system 10 can be configured to evaluate any patient/testing information such as the patient's current BG level, the relationship of the current level to the target range (e.g., is the current BG level above, below, or within the target range), any implemented changes in the protocol, etc. and, in light of this information, determine an appropriate future test time. Stated another way, the system 10 can be configured to classify a patient as high priority or low priority so as to optimize hospital resources. For example, a patient having a high or low current BG level or a patient who recently changed dosages (e.g., increase insulin by 20%) may need to be tested sooner as compared to a patient having a current BG level within the desired range and having no adjustment in dosage. As discussed throughout, the protocol administrator can configure the system 10 to provide for a user override and further scheduling a subsequent test at the user's suggested time. Following this step (Step 308), the system can proceed to a Documentation step (Step 310) wherein the user can document patient status and/or treatment provided. Next, the user may End TGC testing (Step 320).

As indicated above, various steps of the above-described system can be added or removed as desired by the protocol administrator thereby allowing for a user-definable glycemic control protocol. For example, FIG. 9 provides an alternative embodiment wherein the system 10 can include an operator confirmation stage (350) capable of automatically requiring a second operator identification number in response to various criteria (e.g., a change in medication of a certain amount, etc). Looking in detail at FIG. 9, the operator confirmation stage (350), beginning at Step 352, initially can prompt a user for entry of an operator ID (Step 202). Following entry, the system can ask whether the inputted identification number is linked to an authorized and/or certified operator (Step 354). If 'no', the system can reject the user and prevent further use. Alternatively, the system 10 can allow testing to proceed and "flag" any results obtained with a non-validated ("NVO") or non-certified operator ("NCO") tag. If the operator is authorized (system 10 answers 'yes'), the system can proceed to patient testing and determination of any treatment adjustments (Steps 218, 220, 304, 306). Following testing and adjustment determination, the system 10 can be configured to require confirmation (Step 356). If the system 10 is not so configured, the system 10 will proceed as discussed above and thereby exit the confirmation stage (350) (Step 368).

Referring to Step 356, the system 10 can be configured to always require confirmation or may be configured to require confirmation only in certain circumstances such as being dependent upon a patient state (e.g., restart state), based on adjustment parameters (e.g., only when IV insulin is increased or decreased when change is greater than or equal to a set percentage value or set units/h value, or when total a amount of an IV insulin or a bolus exceeds a set units/h value), or based on operator state (e.g., operator flagged as NVO and/or NCO). If the system 10 requires such confirmation, the system 10 can be configured to display adjustment/confirmation information (e.g., prompting a user to acknowledge such adjustments). Next, the system 10 can be configured for entry of a second operator identifier. If not so configured, the system 10 can once again move to Step 368 and exit the confirmation stage (350). If the system 10 is configured to require a second operator identifier, the system 10 can ask for the second identification number (Step 362). Next, like Step 354 above, the system 10 can determine if the second identifier is valid (Step 364). If no, the system 10 can reject the second entry (or once again flag the second operator). If accepted, the system 10 can check to see if the first operator identifier is different from the first confirmation identifier (Step 366). If no, the system can reject the entry. If yes (identifiers are different), the system 10 can end this stage (350) (Step 368).

The following examples and corresponding FIGS. provide a detailed flow chart as well as example user displays screen associated with the various stages. Like above, these are merely provided as examples of the presently disclosed system and are not intended to limit the scope of the present disclosure in any way.

### EXAMPLES

### Example 1

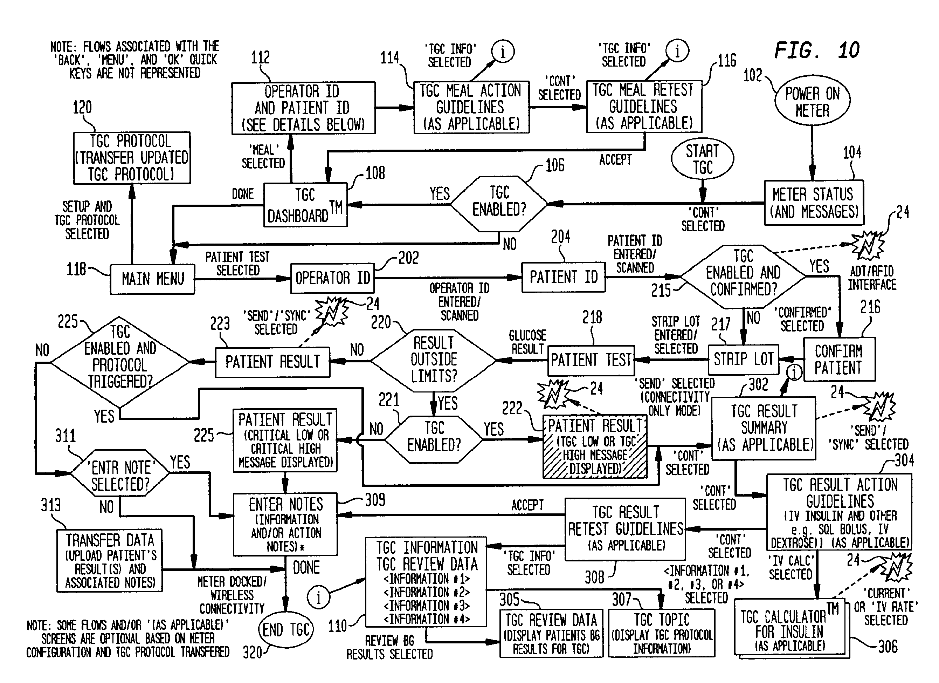

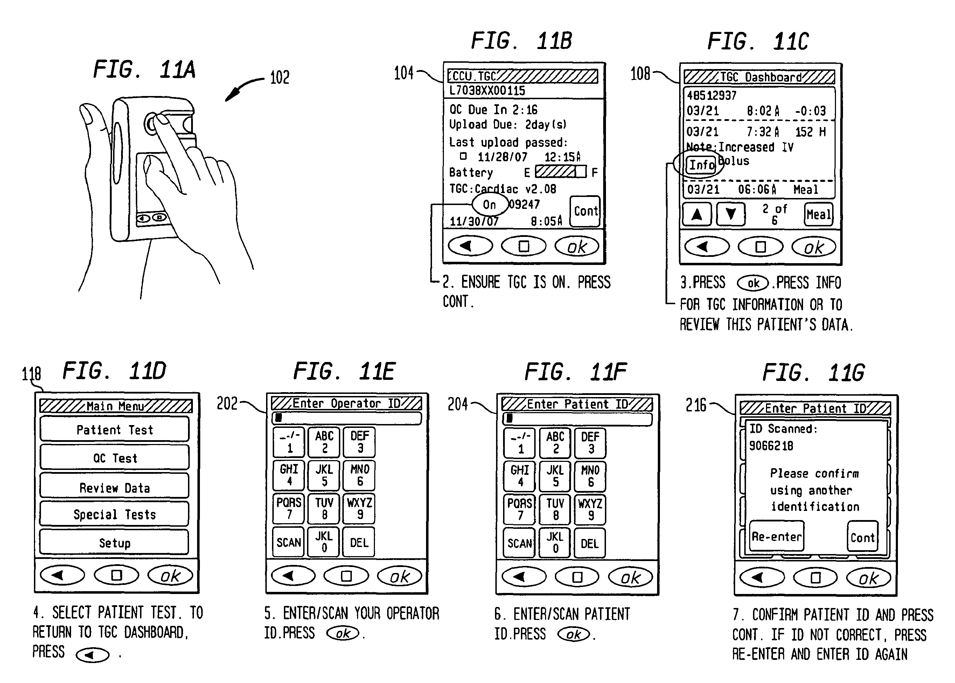

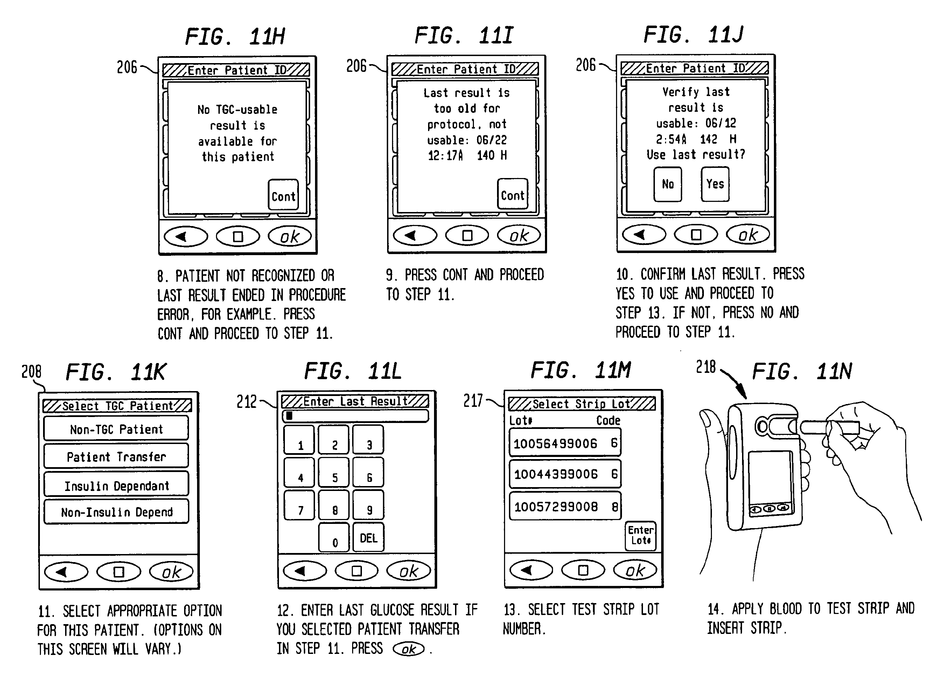

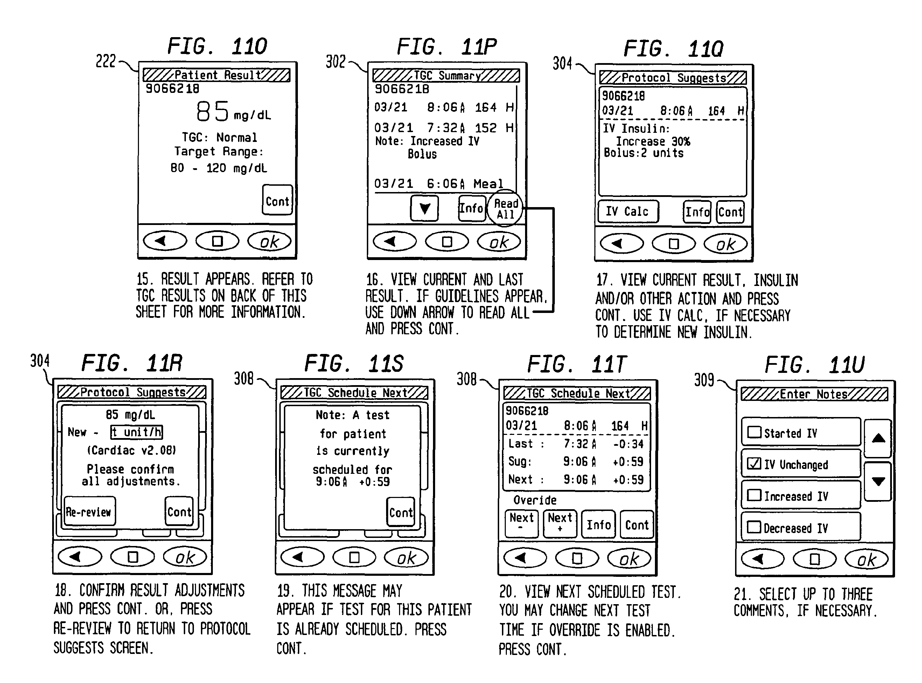

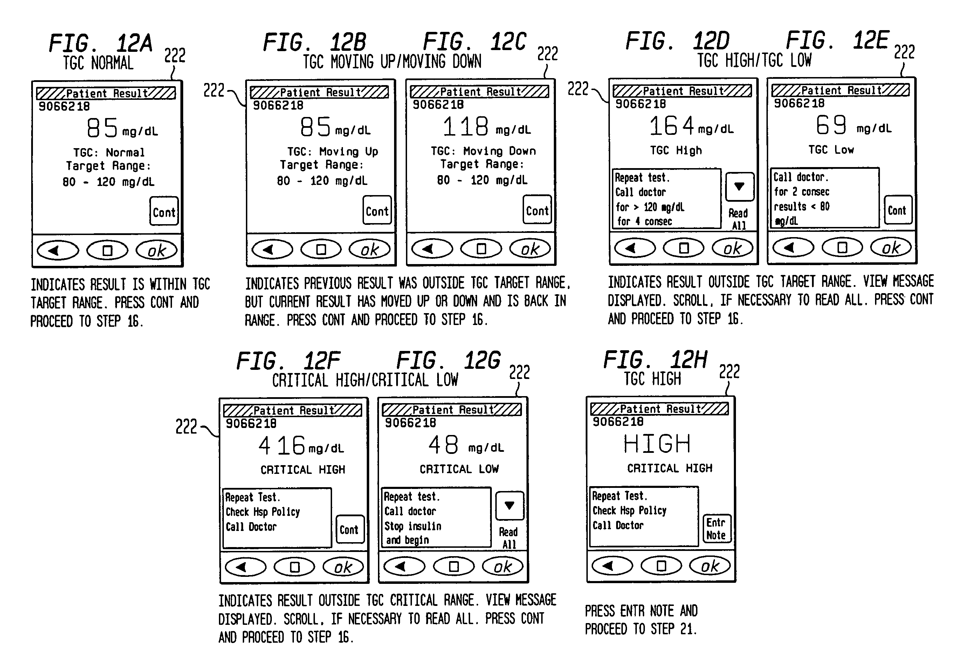

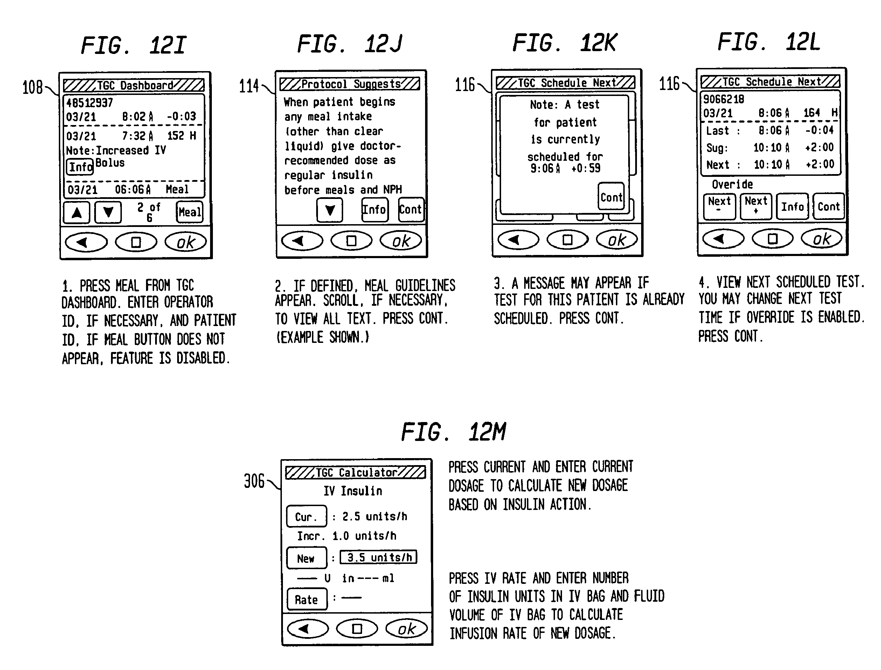

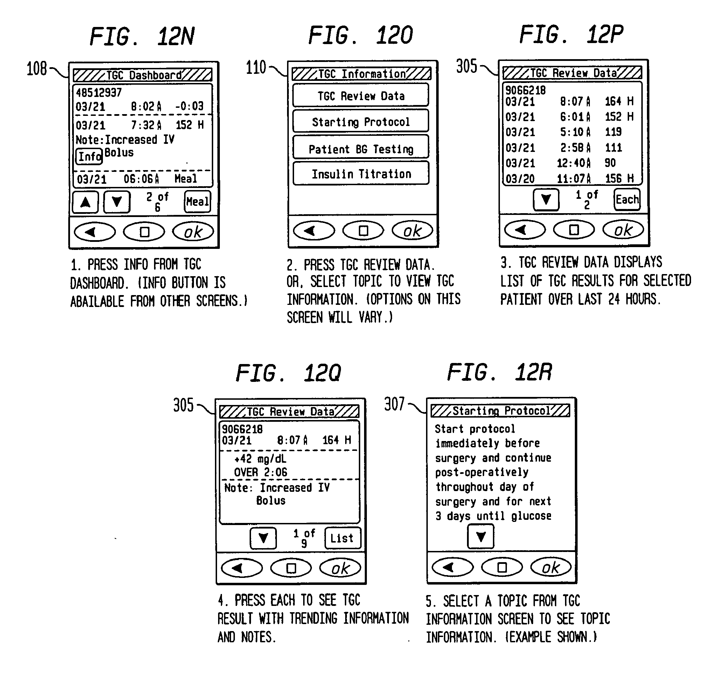

FIG. 10 provides an exemplary embodiment of the presently disclosed system wherein a desired TGC protocol has been loaded onto the OneTouch®Flexx™ with TGC Meter. Furthermore, the example cross-references FIGS. 11A-11U and FIGS. 12A-12R to provide examples of display screens corresponding with the various steps. Again, this discussion is for example purposes only and not meant to limit the scope of the disclosure in any way.

As shown, the system 10 can be activated by powering on the glucose measuring device (Step 102; see FIG. 11A). Once activated, the system 10 can display the Meter Status screen (Step 104; see FIG. 11B). Next, the system 10 can determine whether or not the glucose measuring device 12 is TGC enabled or stated differently, is the glucose measuring device configured to perform TGC testing (Step 106). If 'no', the user can be transferred directly to the Main Menu (Step 118; see FIG. 11D) thereby bypassing various TGC-related steps and allowing the glucose measuring device 12 to continue functioning as a non-TGC device. If the glucose measuring device 12 is TGC-enabled, the user will be directed to the TGC Dashboard (Step 108; see FIG. 11C). While at the TGC Dashboard (Step 108), the user may select a link to 'Meal' information and be directed to Operator/Patient Identification Step (Step 112; See FIGS. 11E and 11F). From here, the user may be directed to a TGC Meal Action Guidelines step (Step 114; see FIG. 12J) and further, to a TGC Meal Retest Guidelines step (Step 116; also, see FIGS. 12K and 12L).

From the TGC Dashboard (Step 108), the user may be directed to the Main Menu (Step 118; see FIG. 11D). From the Main Menu (Step 118), the user may select the TGC Protocol Update Option (Step 120) in order to revise the current protocol (requires protocol administrator as well as Editor and Transfer Tool), or the user may select 'Patient Test'. When selecting 'Patient Test', the user will next be prompted to enter an operator ID (Step 202; See FIG. 11E) and a patient ID (Step 204; FIG. 11F). Once the identification numbers have been entered, the glucose measuring device 12 may once again determine if the glucose measuring device 12 is TGC enabled (Step 215). If 'no', the user is directed to perform the patient test (i.e., a non-TGC-test). If 'yes', the system 10 may prompt the user to confirm the patient ID (Step 216; see FIG. 11G) and then proceed to patient testing.

The OneTouch® Flexx^{™} TGC Meter utilizes a strip test wherein a patient's sample (e.g., blood) is added to a test strip and delivered to the glucose measuring device 12. As such, in this exemplary embodiment, the system 10 may be configured to request a strip lot number (Step 217; see FIG. 11M) followed by applying the patient sample to the test strip, and delivering the test strip to the glucose measuring device 12 in order to perform the patient test (Step 218; see FIG. 11N). After a current BG level is determined, the test result can be transferred to a Calculation Step (Step 220) wherein the current level is compared to the target range to determine whether or not the current level is outside the desired limits. If 'no' (i.e., current level is within the desired range), the result can be transferred to a patient result step (Step 223). Following this flow, the protocol may include another check to verify that TGC is Enabled and Protocol Triggered (Step 225) before transferring the results to a TGC Result Summary Step (Step 302, see FIG. 11P). If the result is outside of the target range (i.e., the answer to Step 220 is 'Yes'), the system 10 can once again verify that TGC is enabled (Step 221). If the answer to this step is 'No', the test information can flow to a non-TGC Patient Result interface (Step 225) and on to an Notes Interface (Step 309) wherein a user can link various qualitative information to the results. Continuing with this flow, the user can then transfer data (Step 313) before exiting the system (Step 320).

Returning to Step 221, if the system is TGC Enabled then all data is transferred and displayed along with any desired qualitative status identifiers in a Patient Results Step (Step 222; various examples of possible results are shown at FIG. 11O and FIGS. 12A-12H). Following a review of the Patient Results (Step 222), the user may be directed to the TGC Result Summary (Step 302, see FIG. 11P), and next to a TGC Result Action Guideline Step (Step 304, see FIGS. 11Q-11R). If a calculation is suggested, the user may select the TGC Calculator (Step 306; see FIG. 12M), which can be configured to calculate and/or implement the suggested protocol change. In addition to utilizing the TGC Calculator (Step 306), the user may once again access the TGC Information screens (Step 110; See FIG. 12O), and may subsequently select TGC Review Data (Step 305; see FIGS. 12P and 12Q) in order to review a patient's previous TGC test results. Alternatively, the user may select TGC Topic (Step 307; see FIG. 12R) in order to review any protocol information provided by the protocol administrator.

Following a review of the various information associated with the TGC Result Action Guidelines (Step 304), the user may select to continue to the TGC Result Retest Guidelines (Step 308, see FIGS 11S-11T) thereby allowing the user to review data regarding when the next test has been scheduled by the system. As shown in FIG. 11T, the system 10 may include an override feature allowing an authorized user to change the scheduled time for the future test. After the future test has been scheduled, the user may be directed to an Enter Notes step (Step 309; see FIG. 11U) wherein the user may enter various notes/comments about the test and or changes to the treatment resulting from the test. Following this step, the user may synchronize the glucose measuring device 12 with hospital network (Step 313) so as to transfer data and proceed to End TGC testing (Step 320). As indicated by the various connectivity symbols 24, the user may synchronize the glucose measuring device 12 with the computer system at any time during the exemplary embodiment.

Various aspects of a method of implementing a glycemic control protocol are also provided herein. In one such aspect, the method includes loading a glycemic control protocol onto a glucose measuring device wherein (similar to above) the glycemic control protocol can be configured to provide at least one treatment step (e.g., an adjustment and/or an action) based at least partially on a patient's blood glucose level measured at a specific time point. Next, the method can include measuring a patient's current blood glucose level with the glucose measuring device, and associating the patient's current blood glucose level with a current time point. The method also can include providing an treatment step which is at least partially determined by the glycemic control protocol. Additionally, the method can further include storing the patient's current blood glucose level, the associated time point, and the treatment step in a database in communication with the glucose measuring device.

In some embodiments, the method can also include comparing the patient's current blood glucose level and current time point with at least one prior blood glucose level determined at a prior time point so as to provide a glucose rate of change, and providing a treatment step which is based at least partially dependent upon the glucose rate of change. In such an embodiment, the treatment step can determined by the glycemic control protocol's interpretation of the rate of change and a prior action. Optionally, the method can also include a step for determining if the patient's current blood glucose level is a valid test result.

In yet another aspect, a method for implementing a glycemic control protocol is provided which includes providing a glucose measuring device and also providing a memory system having a storage means containing a plurality of glycemic control protocols. Additionally, the method can include providing at least one patient, and associating the at least one patient with a patient-specific glycemic control protocol wherein the patient-specific glycemic control protocol can be selected from the plurality of glycemic control protocols contained within the storage means. The method can further include measuring the patient's current blood glucose level, associating the patient's current blood glucose level with a current time point, and providing a treatment step which is determined by the patient-specific glycemic control protocol. Optionally, the method can also include loading the patient-specific glycemic control protocol onto the glucose measuring device.

The method can also include providing a plurality of patients, and further associating each patient of the plurality of patients with a patient-identification tag wherein the patient identification tag includes a storage means configured to store a plurality of patient-specific information. In such an embodiment, the glucose measuring device can be configured to communicate with the patient's patient-identification tag. Also, the method can further include (e.g., following the associating step) modifying the patient-specific protocol thereby providing a user-definable protocol.

One skilled in the art will appreciate further features and advantages of the present disclosure based on the above-described embodiments. Accordingly, the present disclosure is not to be limited by what has been particularly shown and described, except as indicated by the appended claims. All publications and references cited herein are expressly incorporated herein by reference in their entirety.

## Claims

1. A system for implementing a glycemic control protocol, comprising:
a glucose measuring device configured to determine a patient's current blood glucose level at a current time point;
a storage system associated with the glucose measuring device, the storage system configured to store the patient's current blood glucose level and the current time point; and
a processor associated with the glucose measuring device, the processor configured to implement at least one glycemic control protocol, the at least one glycemic control protocol being configured to provide a treatment step, the treatment step being at least partially dependent upon the current blood glucose level and the current time point.

2. The system of claim 1, wherein the treatment step is an adjustment.

3. The system of claim 2, wherein the adjustment is a suggested adjustment.

4. The system of claim 2, wherein the adjustment is a meal adjustment.

5. The system of claim 2, wherein the adjustment is a medication adjustment.

6. The system of claim 5, wherein the medication adjustment is a change in a type of medication being administered to the patient or the medication change is an increase or a decrease in an amount or a dosage of the medication being administered to the patient.

7. The system of claim 6, wherein the medication is any medication capable of manipulating the patient's blood glucose level.

8. The system of claim 1, wherein the at least one glycemic control protocol is configured to provide a plurality of treatment steps, the treatment steps being at least partially dependent upon the current blood glucose level and the current time point.

9. The system of claim 8, wherein the plurality of treatment steps includes a first treatment step and a second treatment step, the first treatment step being an adjustment, the second treatment step being an action, the action being at least partially based upon the adjustment.

10. The system of claim 9, wherein the action is performed automatically in a semiclosed or closed loop manner.

11. The system of claim 1, wherein the treatment step is an action.

12. The system of claim 11, wherein the action includes scheduling a subsequent test to determine a patient's blood glucose level.

13. The system of claim 1, wherein the storage system further stores at least one previous patient blood glucose level and a previous time point associated with the at least one previous patient blood glucose level.

14. The system of claim 13, wherein the storage systems is further configured to store a previous treatment step associated with the previous patient blood glucose level and the previous time point associated with the previous blood glucose level.

15. The system of claim 13, further comprising:
a comparison system associated with the glucose measuring device, the comparison system configured to compare the patient's current blood glucose level and current time point with the at least one previous patient blood glucose level and the previous time point to provide a glucose rate of change.

16. The system of claim 15, wherein the glucose rate of change is an absolute difference, a rate, or a percent.

17. The system of claim 15, wherein the treatment step is at least partially dependent upon the glucose rate of change.

18. The system of claim 1, wherein the glucose measuring device is configured to distinguish between a plurality of patients such that each patient can be correlated with a corresponding glycemic control protocol.

19. The system of claim 18, wherein the glucose measuring device is configured to communicate with a patient-specific database located on a patient-specific RFID tag.

20. The system of claim 1, wherein the storage system is located within the glucose measuring device.

21. The system of claim 20, wherein the processor is located within the glucose measuring device.

22. The system of claim 1, wherein the glucose measuring device is a hand-held device.

23. The system of claim 1, further comprising:
a user interface in communication with the glucose measuring device, the user interface configured to display a patient status, the patient status at least partially dependent on the current blood glucose level and the current time point.

24. A system for implementing a user-definable glycemic control protocol, comprising:
a glucose measuring device configured to determine a patient's blood glucose level at a current time point;
a memory system having a storage means, the memory system being configured to communicate with the glucose measuring device, the storage means configured to store the patient's current blood glucose level and the current time point; and
a processor configured to communicate with the glucose measuring device, the processor further configured to implement a user-definable protocol, the use-definable protocol configured to determine a treatment step based at least partially on the patient's current blood glucose level and the current time point.

25. The system of claim 24, wherein the memory system is located within the glucose measuring device.

26. The system of claim 25, wherein the processor is located within the glucose measuring device.

27. The system of claim 24, further comprising:
an editor in communication with the processor, the editor configured to modify the glycemic control protocol in response to instructions from a protocol administrator.

28. The system of claim 24, wherein the glucose measuring device is configured to receive an operator identifier prior to use, the storage means being configured to associate the operator identifier with the current blood glucose level and the current time point.

29. The system of claim 28, where the glucose measuring device is configured to require a second operator identifier associated with a second operator prior to use.

30. A method of implementing a glycemic control protocol, comprising:
loading a glycemic control protocol onto a glucose measuring device, the glycemic control protocol configured to provide a treatment step based at least partially on a patient's blood glucose level measured at a specific time point;
measuring a patient's current blood glucose level with the glucose measuring device;
associating the patient's current blood glucose level with a current time point; and providing an treatment step which is at least partially determined by the glycemic control protocol.

31. A method for implementing a glycemic control protocol, comprising:
providing a glucose measuring device;
providing a memory system having a storage means containing a plurality of glycemic control protocols;
providing at least one patient;
associating the at least one patient with a patient-specific glycemic control protocol, the patient-specific glycemic control protocol selected from the plurality of glycemic control protocols contained within the storage means;
measuring the patient's current blood glucose level;
associating the patient's current blood glucose level with a current time point; and
providing a treatment step which is determined by the patient-specific glycemic control protocol.
